(19) **Europäisches Patentamt**
**European Patent Office**
**Office européen des brevets**

(11) **EP 2 077 753 B1**

(12) **EUROPEAN PATENT SPECIFICATION**

(45) Date of publication and mention
of the grant of the patent:
**19.12.2012 Bulletin 2012/51**

(51) Int Cl.:
***A61B 5/103*** (2006.01)     ***A61B 5/00*** (2006.01)

(21) Application number: **07814824.4**

(86) International application number:
**PCT/US2007/078254**

(22) Date of filing: **12.09.2007**

(87) International publication number:
**WO 2008/033909 (20.03.2008 Gazette 2008/12)**

(54) **APPARATUS, PROBE AND METHOD FOR PROVIDING DEPTH ASSESSMENT IN AN ANATOMICAL STRUCTURE**

VORRICHTUNG, SONDE UND VERFAHREN ZUR TIEFENBEURTEILUNG BEI EINER ANATOMISCHEN STRUKTUR

APPAREIL, SONDE ET PROCEDE DESTINES A EFFECTUER DES EVALUATIONS DE PROFONDEUR DANS UNE STRUCTURE ANATOMIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.09.2006 US 844302 P**

(43) Date of publication of application:
**15.07.2009 Bulletin 2009/29**

(73) Proprietor: **The General Hospital Corporation Boston, MA 02114 (US)**

(72) Inventors:
• **TEARNEY, Guillermo J.**
  **Cambridge, MA 02139 (US)**
• **BOUMA, Brett E.**
  **Quincy, MA 02171 (US)**
• **NADKARNI, Seemantini K.**
  **Boston, MA 02114 (US)**

(74) Representative: **Maiwald Patentanwalts GmbH Elisenhof**
**Elisenstrasse 3**
**80335 München (DE)**

(56) References cited:
**WO-A-99/05487     US-A1- 2006 155 193**

• **SADHWANI A ET AL: "DETERMINATION OF TEFLON THICKNESS WITH LASER SPECKLE. I. POTENTIAL FOR BURN DEPTH DIAGNOSIS" APPLIED OPTICS, OSA, OPTICAL SOCIETY OF AMERICA, WASHINGTON, DC, US, vol. 35, no. 28, 1 October 1996 (1996-10-01), pages 5727-5735, XP000629798 ISSN: 0003-6935**
• **STEWART ET AL: "A comparison of two laser-based methods for determination of burn scar perfusion: Laser Doppler versus laser speckle imaging" BURNS, BUTTERWORTH HEINEMANN, GB, vol. 31, no. 6, September 2005 (2005-09), pages 744-752, XP005046306 ISSN: 0305-4179**
• **TEARNEY G J ET AL: "Atherosclerotic plaque characterization by spatial and temporal speckle pattern analysis" CONFERENCE ON LASERS AND ELECTRO-OPTICS. (CLEO 2001). TECHNICAL DIGEST. POSTCONFERENCE EDITION. BALTIMORE, MD, MAY 6-11, 2001, TRENDS IN OPTICS AND PHOTONICS. (TOPS), US, WASHINGTON, WA : OSA, US, vol. VOL. 56, 6 May 2001 (2001-05-06), pages 307-308, XP010559872 ISBN: 1-55752-662-1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

## FIELD OF THE INVENTION

The present invention relates to apparatus, probes and methods for obtaining information associated with an anatomical structure, and more particularly to such apparatus, probes and methods which can provide an assessment of such anatomical structure at different depth, and e.g., to review the information for damage therein include burn damage.

## BACKGROUND INFORMATION

Early, rapid assessment and treatment of burn wounds can be life saving. Burn depth Has been assessed by a clinical inspection, which may have an accuracy of only approximately 50% even when reviewed by an experienced bum surgeon or physician. While certain methods have been proposed for evaluating burn injuries, there is still no widely accepted diagnostic method or system which can be used non-invasively determining burn depth in patients. A number of techniques and/device have been described for determining burn depth, including exogenous dye fluorescence, radioactive isotopes, dye absorption, high frequency ultrasound, laser Doppler flowmetry and imaging, and reflectance spectroscopy. At least some of these techniques/devices may have significant disadvantages, including poor accuracy in the early period following a burn injury, a prolonged measurement period, the potential for patient toxicity, etc. Moreover, many of such conventional devices can be cumbersome and expensive and, as a result, not likely to be of use to a general practitioners, emergency room physicians, or medical personnel in the field.

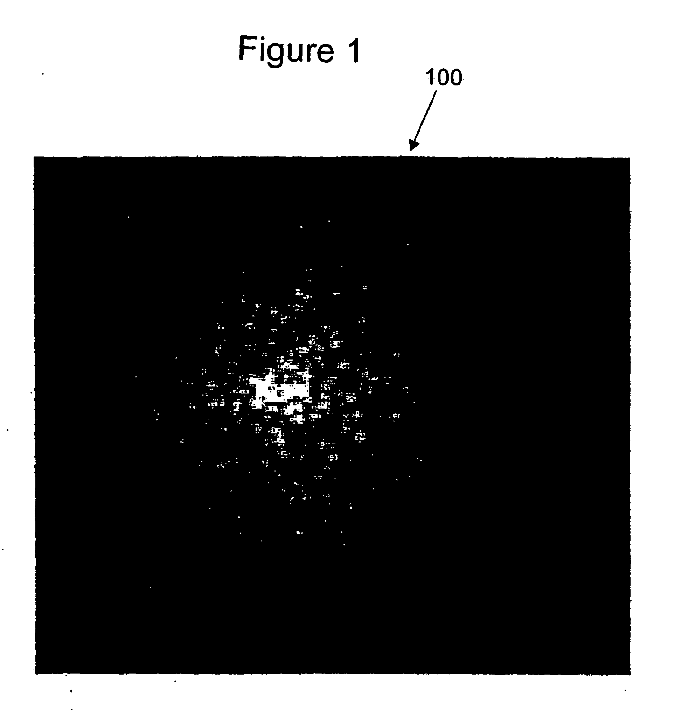

Following thermal injury, an external portion of the skin generally becomes irreversibly damaged (eschar) and likely loses its blood supply. If the burn depth (e.g., thickness of the eschar layer) does not involve the basal portions of hair follicles and sweat ducts, the burn may heal by re-population of the skin surface by epithelial cells originating from these skin appendages, located in the highly vascular dermis. The anatomy of a burn can therefore be modeled as a two-layered medium consisting of an avascular layer (eschar) overlying a vascular layer (viable tissue) (as shown in Figure 1). A device capable of determining the thickness of the avascular layer may provide an estimate of the burn depth, predict the likelihood of epidermal re-epithelialization, and identify deep burn wounds for excision and grafting at an early stage.

Stewart CJ et Al in A comparison of two laser based methods for determination of burn scar perfusion: Laser Dopple versus laser speckel imaging published by Elsevier in 2005 discloses a device according to the preamble of claim 1.

Thus, there may be a need to overcome at least some of the deficiencies associated with the conventional arrangements and methods described above.

## OBJECTS AND SUMMARY OF EXEMPLARY EMBODIMENTS OF THE INVENTION

To address and/or overcome the above-described problems and/or deficiencies as well as other deficiencies, exemplary embodiments of apparatus, which can provide an assessment of such anatomical structure at different depth, and e.g., to review the information for damage therein include burn damage can be facilitated.

The invention is defined by the appended claim. According to one exemplary embodiment of the present invention, apparatus, can be provided for at least such reasons and others. In particular, an exemplary embodiment of apparatus can be provided for obtaining information regarding at least one portion of an anatomical structure. For example, using at least one first arrangement, it is possible to forward at least one first electromagnetic radiation to the at least one portion. In addition, using at least one second arrangement, it is possible to detect at least one second electromagnetic radiation from the sample, the second electromagnetic radiation being related to the first electromagnetic radiation. The second arrangement can be used to obtain data associated with the second electromagnetic radiation at a plurality of integration times.

Further, with at least one third arrangement, it is possible to determine at least one motion characteristic as a function of depth within the portion using the second electromagnetic radiation. The motion characteristic can be determined by obtaining data associated with the second electromagnetic radiation. The data can be speckle data detected by the second arrangement. In addition, using the third arrangement, it is possible to determine optical properties of the portion based on the data. Furthermore, with a fourth arrangement, it is possible to receive the first electromagnetic radiation and generate a plurality of third electromagnetic radiations to irradiate multiple points on the portion. The second arrangement can be used to detect the second electromagnetic radiation from the sample based on the third electromagnetic radiations, and the third arrangement can also be used to determine a plurality of motion characteristics as a function of depth for a plurality of location of the at least one portion as a function of the second electromagnetic radiation.

These and other objects, features and advantages of the present invention will become apparent upon reading the following detailed description of embodiments of the invention, when taken in conjunction with the appended claims.

## BRIEF DESCRIPTION OF THE DRAWINGS

Further objects, features and advantages of the invention will become apparent from the following detailed description taken in conjunction with the accompanying figures showing illustrative embodiments of the invention, in which:

Figure 1 is an exemplary speckle image illustrating a granular interference pattern that may be observed when illuminating tissue with coherent light;

Figure 2 is a schematic diagram of a burn model in accordance with an exemplary embodiment of the present invention;

Figure 3 is a flow diagram of a first exemplary embodiment of a method according to the present invention;

Figure 4 is a flow diagram of a second exemplary embodiment of a method according to the present invention;

Figure 5 is schematic diagram of an exemplary embodiment of an apparatus according to the present invention;

Figure 6 is a block diagram of an optical arrangement according to an exemplary embodiment of the present invention which can be used to obtain time-integrated laser speckle images;

Figures 7(a)-7(d) are series of speckle images obtained from an exemplary embodiment of a Teflon scattering phantom moving at, e.g., 5 mm/sec.;

Figure 8 is an exemplary graph of a relative velocity measured by an exemplary embodiment of a time-integrated speckle apparatus according to the present invention as a function of the actual velocity;

Figure 9 is a diagram of an exemplary embodiment of a miniature platform for conducting time-integrated laser speckle imaging ("LSI") procedures in accordance with the present invention;

Figures 10(a) and 10(b) are exemplary illustrations of photons propagations through the depths of an anatomical structure and the decorrelation times associated therewith;

Figure 11 is a graph of exemplary radial photon probabilities measured experimentally for a fibrous plaque compared to theoretical radial photon probabilities calculated from an exemplary diffusion model for a semi-infinite homogenous tissue;

Figure 12 is a graph of exemplary results of Monte Carlo simulations in which the radially-resolved photon penetration depth is provided as a function of distance from an illumination location; and

Figure 13 is a graph of an exemplary penetration depth provided as a function of an average fibrous cap thickness measured from histology.

Throughout the figures, the same reference numerals and characters, unless otherwise stated, are used to denote like features, elements, components or portions of the illustrated embodiments. Moreover, while the subject invention will now be described in detail with reference to the figures, it is done so in connection with the illustrative embodiments. It is intended that changes and modifications can be made to the described embodiments without departing from the true scope and spirit of the subject invention as defined by the appended claims.

## DETAILED DESCRIPTION OF EXEMPLARY EMBODIMENTS

### General Discussion

One exemplary non-invasive method for measuring eschar thickness is a spatial and temporal analysis of laser speckle patterns. Related materials for this exemplary method is described in Sadhwani, A., et al., Appl. Opt. 35(28): 5727-5735, 1996, Nadkarni, S. et al., Journal of Biomedical Optics 11(2):021006, and U.S. Patent Application Serial No. 10/016,244 filed October 30, 2001.

For example, speckle is a granular interference pattern that may be observed when illuminating tissue with coherent light (as shown in the example of Figure 1). The speckle pattern formed at the surface of a burn can be determined by a microscopic structure of the skin, and may be exquisitely sensitive to a particle motion within the tissue. As a result, as speckle patterns formed from light that has traversed vascular tissue may be temporally modulated. In contrast, the speckle patterns formed from light that has only traversed avascular (eschar) regions generally remain constant over time. For example, when tissue is illuminated with a narrow diameter beam, photons propagating deeper into the tissue have a higher probability of exiting the tissue further away from the source entry point. A two-dimensional image of the laser speckle pattern formed on the surface of a burn should therefore contain a central region with a static speckle pattern and a surrounding region with a modulated speckle pattern. A measurement of the radius of the static region, along with knowledge of the optical properties of the eschar, should allow a determination of the thickness of the eschar. In addition, a temporal correlation of the modulated speckle pattern may provide an estimate of tissue perfusion in the viable layer.

There are different exemplary ways to measure speckle pattern fluctuations, e.g., time varying measurements and time-integrated measurements. Time-varying speckle can be described by the characteristic time constant, $T_d$, over which the speckle pattern decorrelates. A static sample that undergoes no Brownian motion or flow likely has a static speckle pattern, resulting in a high decorrelation time constant. Time-averaged speckle can be characterized by a quantity called the speckle contrast, defined as $\sigma(\rho,T)/<I(\rho,T)>$, where T is the integration time of the speckle pattern (the exposure time of the camera or duration of the light source illumination) and p is the distance from the incident beam. In a static sample a high contrast speckle image is observed. When scatterers in the sample are in motion, a blurred speckle pattern likely results, thus decreasing the speckle contrast. For a time-integrated speckle pattern analysis, the decorrelation time constant can be obtained by first determining the decorrelation function by solving the following exemplary equation:

$$\min\left|c(T)-\sqrt{\frac{1}{T}\int_0^T C(t)dt}\right|, \qquad \text{Eq. (1)}$$

where T is the integration time, $c(T)$ is the measured time-integrated contrast, and $C(t)$ is the decorrelation function.

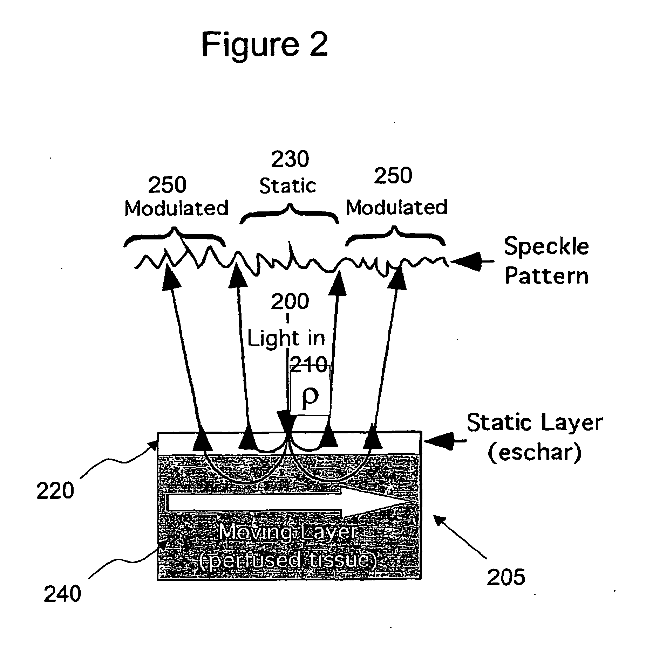

An exemplary spatio-temporal analysis of time-integrated or time-resolved speckle patterns formed by point illumination of tissue can be utilized to obtain depth-resolved information Monte Carlo simulations and analytic solutions to the radiative transport equation have shown that light diffuses radially outward from the illumination location to emerge at a radius proportional to the depth it has penetrated (as illustrated in Figure 2). For example, as shown in Figure 2, light 200 is input into a sample 205. Therefore, the analysis of the speckle modulation as a function of radial distance from the source location, p 210, can provides certain information regarding tissue perfusion as a function of depth within the sample 205. Because burned tissue or eschar 220 is necrotic, it likely has a reduced blood flow motion, and thus a temporally static speckle pattern 230. Conversely, speckle formed from light that has traversed vascular tissue 240 is likely temporally modulated 250. The speckle pattern resulting from a burn injury should therefore exhibit a high speckle decorrelation time constant within a radius corresponding to the depth of the necrotic tissue. Outside of this radius, light propagates through the perfused tissue 205, resulting in a low time constant in these areas. The radii at which these time constant transitions generally occur and the depths of penetration are likely related by the optical properties of tissue. Advantageously, these optical properties may be measured by using the time-averaged speckle pattern via an exemplary technique of diffuse reflectance spectrophotometry. The velocity profiles as a function of depth can be obtained by using, e.g., measured optical parameters and time constant radius. These depth-resolved velocity profiles may be determined using an exemplary model for the optical transport of light in scattering media, such as Diffusion theory or Monte Carlo modeling.

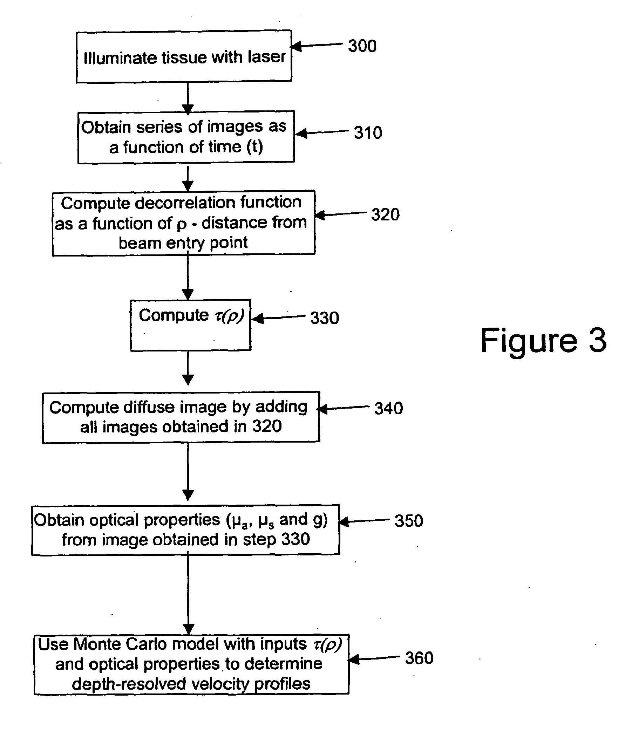

Figure 3 shows a first exemplary embodiment of a procedure or method for recovering depth resolved velocity distributions using time-resolved speckle according to the present invention. For example, light with temporal coherence is input into the tissue of interest in step 300. A CCD, CMOS camera or the like can obtain a series of images as a function of time in step 310. In step 320, a decorrelation curve can be determined from the series of images by using temporal decorrelation of the first image with the remainder of the images in either the time domain or the Fourier domain. In one exemplary embodiment, multiple decorrelation images may be obtained as an additional function of the distance from the beam entry point using m x n window, where the window may be centered at p, i.e., the distance from the beam illumination point. The time constant $\tau(\rho)$ may be determined as a function of $\rho$ in step 330 using a fit to a multiple exponential procedure or fit to a pre-determined flow distribution probability distribution function. To determine the depth-dependent flow, the optical properties $\mu_s$, $\mu_a$, and g for the sample can also be measured. This may be accomplished in step 350 via a variety of ways known in the art. In one exemplary embodiment, the optical properties may be determined from the diffuse image (step 340).

The diffuse image may be obtained by summing up the time-resolved speckle patterns and/or by using the longest time-integrated speckle pattern. The optical parameters may be reconstructed therein using a diffuse reflectance spectrophotometry procedure, where the radial decay of the diffuse reflectance profile may be utilized in conjunction with a diffusion theory to recover the optical properties. Additionally, the total reflectance may be utilized to recover the albedo. Following a measurement of the optical properties, these parameters and $\tau\rho$ may be input into a Monte Carlo or Diffusion theory procedure and the flow velocities as a function of depth may be determined using a forward solution procedure or by using a look-up table of previously determined Monte Carlo simulations (step 360). This exemplary procedure is described in detail in Nadkarni, S. et al., Journal of Biomedical Optics 11 (2):021006.

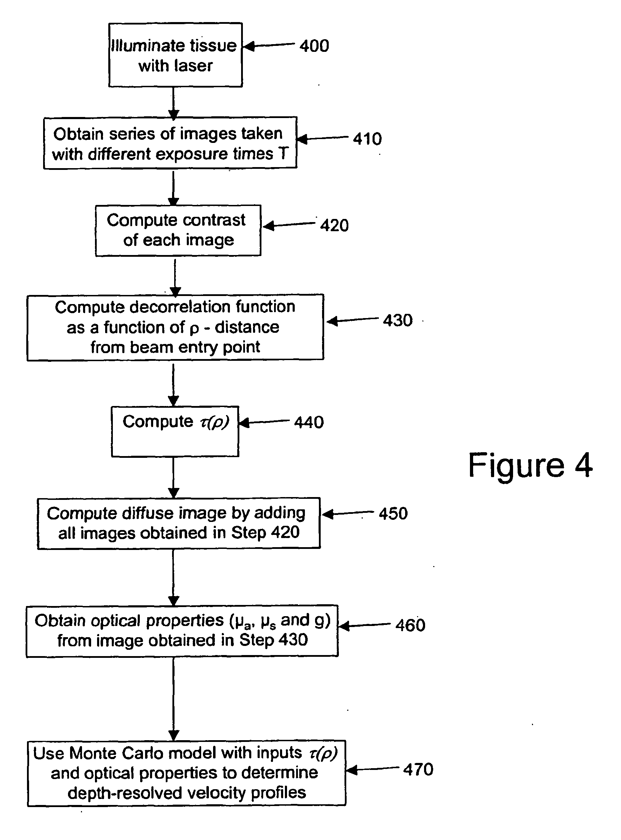

Figure 4 illustrates an exemplary embodiment of a procedure or method for recovering depth-resolved velocity distributions using time-integrated speckle according to the present invention. This exemplary time integrated speckle procedure/method differs from the time-resolved speckle as follows: a) in step 410, speckle patterns are obtained as a function of integration time of the CMOS camera or CCD camera; and b) contrast is obtained for each image in step 420, and the decorrelation function is then determined via Eq. 1 in step 430.

A use of an exemplary embodiment of a laser speckle analysis procedure for estimating burn depth may have advantages over other proposed methods. For example, measurements can be made non-invasively and without an addition of exogenous compounds. An observation of laser speckle may use a simple optical source, such as a laser diode (laser pointer) and a CCD or CMOS camera. Such equipment is likely very inexpensive and readily available. Moreover, these exemplary devices are likely extremely small in size, can be battery powered, and may be easily configured as a pen-sized hand-held probe. Wireless operation can also be implemented since the miniature battery-powered RF transmitters developed for the surveillance industry may be used to transmit speckle images. The cost-effectiveness and portability of laser speckle analysis potentially can makes this exemplary technology accessible to a large number of personnel involved with, e.g., management of critically burned patients.

## Details of Exemplary Embodiments

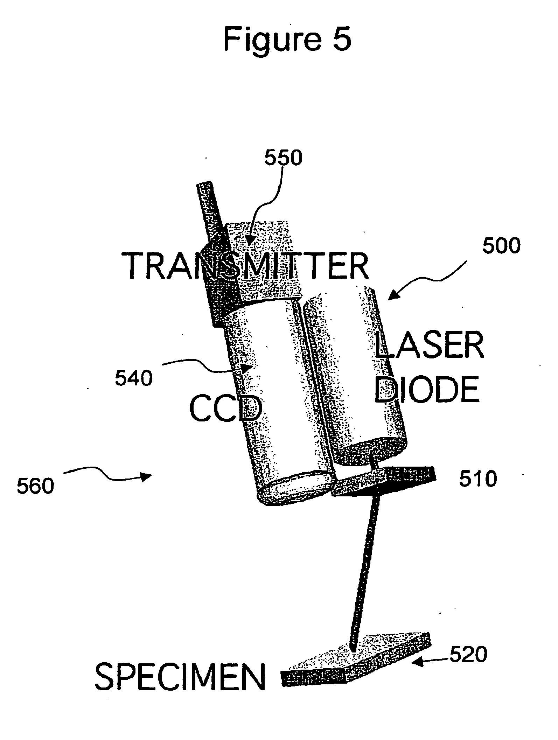

A schematic diagram of one exemplary embodiment of an apparatus according to the present invention is shown in Figure 5. an exemplary footprint of an exemplary embodiment of a hand-held laser speckle burn probe according to the present invention is likely similar to that of the exemplary embodiment of the apparatus as shown in Figure 5.

*Illumination.* The exemplary embodiment of a probe/apparatus according to the present invention can include a battery powered laser diode for illumination of a sample 520. Alternatively or in addition, laser sources such as Helium Neon lasers, solid-state lasers, and LED's may be used. One exemplary characteristic of the light source can be that the temporal coherence length can be effective for obtaining high contrast speckle patterns over the burn eschar depth. As a result, the coherence length may be, e.g., at least 1 mm and preferably greater than 1 cm. In another exemplary embodiment, the coherence length may be varied and the speckle contrast may be used to determine the eschar layer thickness. In yet another exemplary embodiment, the coherence length may be, e.g., less than 1 cm and the speckle mean and variance may be used to determine the layer thickness. For time integrated laser speckle pattern imaging, a pulsed laser diode can be utilized to control the integration time, even though the camera integration time may remain fixed. For this exemplary embodiment, the pulse duration may control the integration time. In still another exemplary embodiment, the power of the laser may be modified for every pulse so that the integrated intensity on the detector is maintained substantially constant for each of the integration times.

*Detector.* In the exemplary embodiment of the apparatus/probe 560 according to the present invention as shown in Figure 5, a battery powered wireless one- or two-dimensional CCD or CMOS camera 540 can be used to acquire the speckle images. The camera 540 may communicate with, e.g., an embedded or separate processor 550 for analysis of the speckle images. Alternatively or in addition, an RF transmitter 550 may transmit video data to a processor distant from the exemplary apparatus/probe 560. Frame rates ranging from, e.g., about 30 - 1000 images per second can be evaluated for assessment of the velocity of the moving layer. In this exemplary manner, tissue perfusion and eschar depth can be estimated. In another exemplary embodiment, images will be acquired using different integration times by either pulsing a laser (or another source of electro-magnetic radiation) 500 with different pulse durations or by changing the exposure time of the camera 540. In order to keep the intensities similar for the different integration times, the power of the laser 500 may be modified so that the integrated power is substantially the same for each integration time. Alternatively, the gain of the detector/CCD/CMOS (e.g., camera) 540 may be modified so that the signal intensity is substantially similar for each of the integration times.

*Source-detector relationships.* A variety of exemplary probe configurations can be utilized, including different illumination wavelengths, source coherence lengths, source-detection angles, and source-detection polarization orientations. An exemplary embodiment of an arrangement for illuminating the field of view with multiple points to reconstruct burn depth estimates over a two-dimensional area can also be used. Passive optical elements capable of performing this exemplary operation can include diffraction gratings, multiple-beam beam splitters, and holographic optical elements and pattern generators. Depth data from multiple, simultaneously illuminated discrete points can be interpolated to create a two-dimensional map of the burn depth. For example, a RF receiver connected to a laptop computer may be used for data acquisition, image processing, and determination of burn depth estimates. Alternatively or in addition, an embedded processor may be utilized to reconstruct the depth-resolved velocity distributions.

*Exemplary Image processing procedures.* Exemplary embodiments of image processing procedures for determining the size of static speckle patterns, such as adaptive local variance determination using, e.g., automatic thresholding, can be used. Non-invasive optical property measurement may be accomplished by diffuse reflective spectrophotometry (as described in Farrell, T.J. et. al., Med. Phys. 19:879, 1992), or by a determination of the second-order statistics of the speckle pattern probability distribution function (as described in Thompson, C.A. et. al., Appl. Opt. 36:3726, 1997). The static speckle size in combination with optical property estimates can provide, e.g., eschar layer thickness and burn depth. Perfusion estimation can be computed by a temporal speckle pattern correlation procedure in the Fourier domain. An exemplary embodiment of a procedure for recovering thickness of static layer over a non-static layer is described in Nadkami, S. et al., Journal of Biomedical Optics 11 (2):021006.

## Examples

An exemplary embodiment of a time-resolved speckle measurement system can be provided which can be utilized to measure the optical properties of tissue and determine depth-resolved tissue motion and flow. Since time-resolved speckle generally uses high-speed CCD cameras, which are likely expensive and relatively large, it is advantageous to obtain these measurements with a time-integrated system. In contrast to the time resolved speckle imaging exemplary procedures, the time integrated speckle measurement exemplary procedures can be conducted with inexpensive and small instrumentation, making it possible and even likely for incorporation thereof in small devices such as a hand-held device.

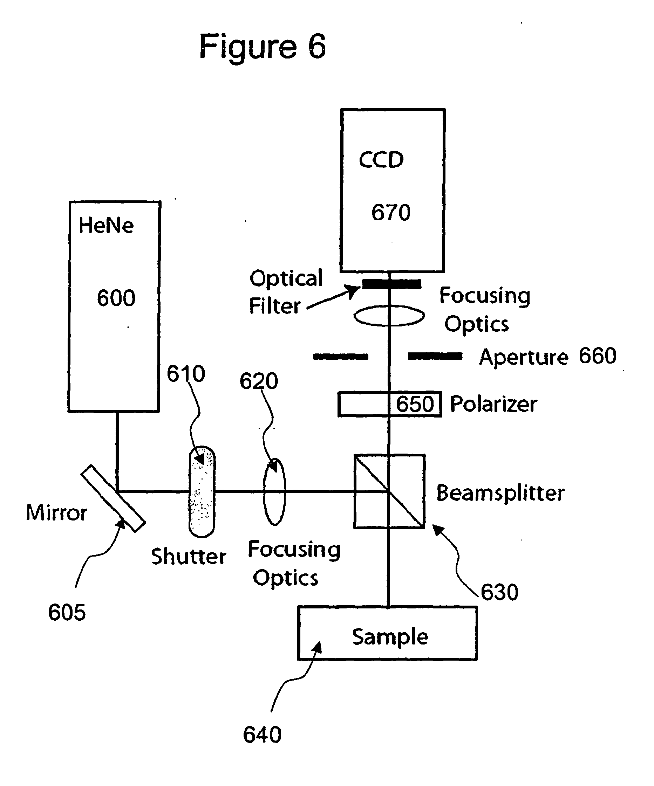

A block diagram of an exemplary embodiment of an arrangement according to the present invention which can be used to obtain time-integrated laser speckle images is illustrated in Figure 6. For example, a polarized helium-neon laser ($\lambda$ = 632nm, 4mW) 600 illuminates a shutter 610 that can control both the light throughput and exposure time. Coherent laser light may then be focused by a lens 620 and transmitted through a beamsplitter 630. The focused spot diameter on the sample 640 may be, e.g., about 100 $\mu$m. Light remitted from the sample can be transmitted through a polarizer 650 and aperture 660 to ensure cross-polarized detection and control the detected speckle size. The speckle pattern can be detected by a compact CCD camera (e.g., JAI-CV11, 30fps, 640x480 pixels) 670. The exemplary embodiment of a time-integrated laser speckle measurement device may be sufficiently small and light to be held in one hand.

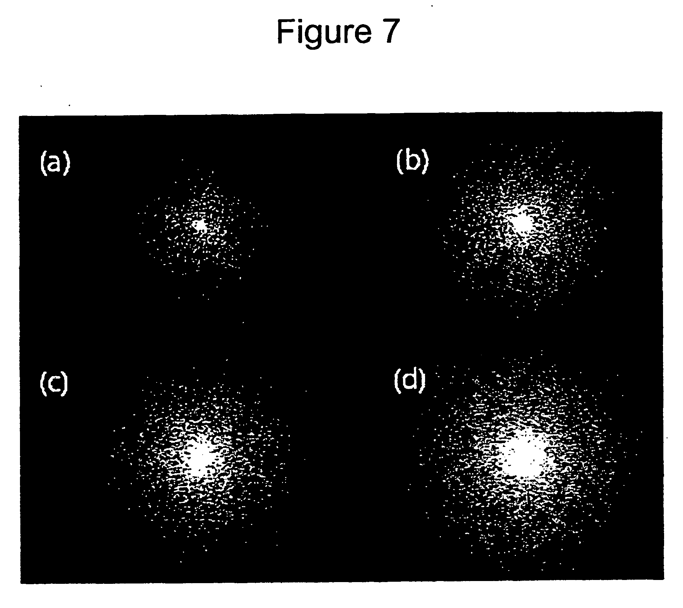

An exemplary procedure can be used to automatically acquire a series of time-integrated speckle images and conduct analysis in real time. Speckle images may be acquired over integration periods ranging from about 5.2ms to about 20.2 ms. Exemplary decorrelation time constants (e.g., inversely proportional to blood flow velocity) may be determined by computing the contrast in speckle images obtained at different integration times. Figures 7(a)-7(d) depict a series of speckle images that can be obtained from an exemplary Teflon scattering phantom, moving at about 5 mm/s. Speckle images are shown in Figures 7(a)-7(d) are provided for exposure times of 5.2 (700), 10.2 (710), 15.2 (720) and 20.2 (730) ms, respectively. Contrast and exposure time were inversely related.

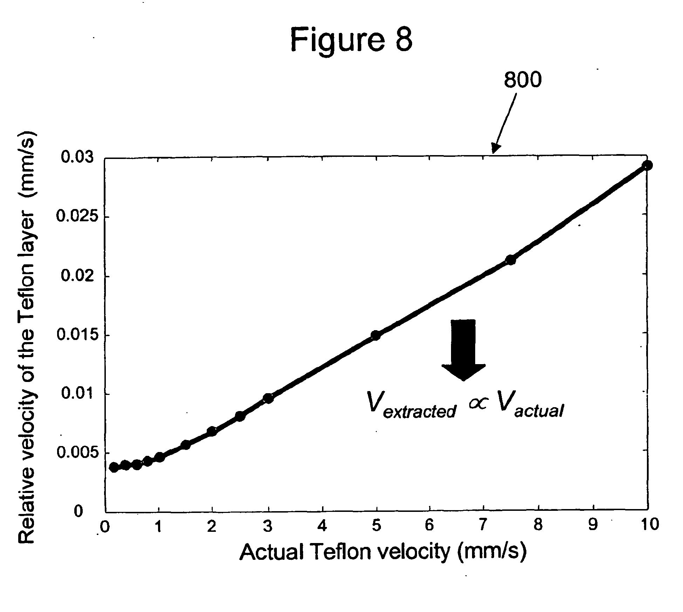

This exemplary embodiment of the system according to the present invention has been used to validate time-integrated speckle for velocity measurements. Scattering phantoms can be moved at a range of velocities (e.g., about 0.2 mm/s - 10 mm/s), which may approximate the range of blood flow rates expected in the skin. Figure 8 shows a graph 800 of the relative velocity measured by our time-integrated speckle apparatus as a function of actual velocity. The actual velocity shown in graph 800 differs from the speckle pattern velocity by a scaling constant. A linear relationship can be observed between the velocities measure by time-integrated speckle pattern and the true velocities of the phantoms (e.g., R=0.99, p<0.001). These results may indicate that time-integrated speckle pattern analysis can be utilized to recover the velocities of blood flow in tissue.

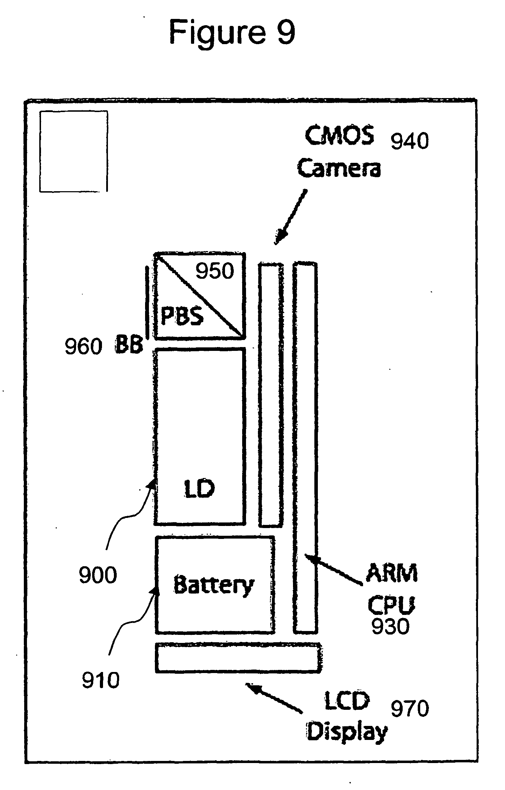

Thus, an exemplary embodiment of, e.g., a miniature platform for conducting time-integrated LSI can be provided, a schematic of an exemplary embodiment of the system providing the same is shown in Figure 9. For example, the exemplary embodiment of the system illustrated in Figure 9 can include a laser diode 900, imaging optics, a miniature ARM computer 930, a CMOS camera 940, and Li-ion battery 910. The laser diode 900 can provides, e.g., about 5 mW of linearly polarized light at 650 nm. In order to provide images with different integration times and constant integrated power, TTL pulses that are input into leads on the laser diode 900 housing may control the power and pulse-duration. The laser output can be directed through a miniature lens in the laser housing to a polarizing beam splitter (PBS) 950 that can be placed in contact with the tissue surface. Light remitted from the tissue may pass back through the PBS 950, and imaged by a miniature CMOS camera 940.

A synchronization of the camera and the pulse integration time and power can be provided by a miniature CPU 930. The miniature CPU 930 can have enough computational power to facilitate all laser speckle imaging processing to be conducted in real time. When activated, the CPU 930 can capture laser speckle images with integration times ranging from 1-30 ms, at 1 ms increments. The image data may be stored on a miniature SD-card that resides on the miniature computer 930. According to one exemplary embodiment of the present invention, the CMOS camera 940 driver and software can be provided in the miniature computer 930 and images can be acquired. It is also possible to use other exemplary procedures to reconstruct depth-resolved velocity profiles from the image data and constructing a mechanical housing for the device. Further, it is possible to incorporate a small LCD display 970 that may provide a map of depth-dependent flow distributions.

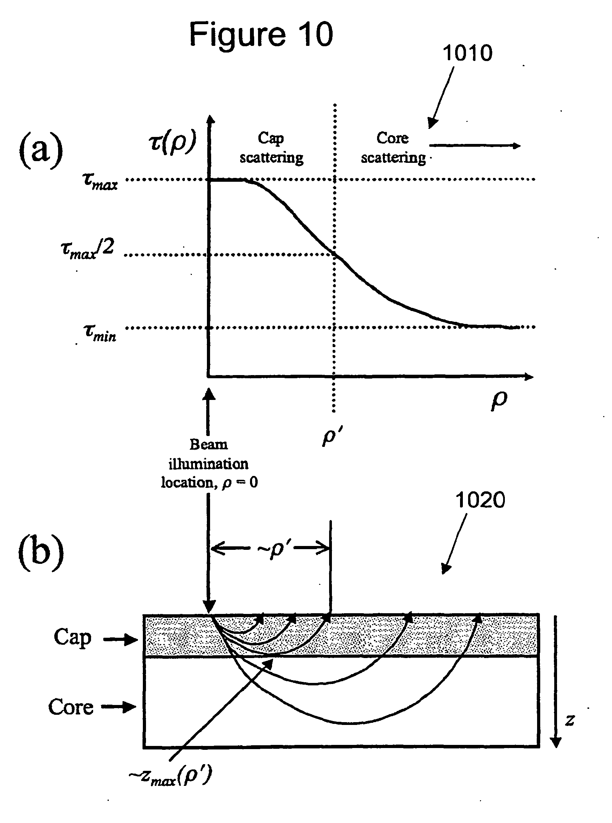

An exemplary embodiment of a procedure according to the present invention that can be used to reconstruct depth-resolved motion in samples from LSI may detect the thickness of fibrous caps in a certain type of arterial atherosclerotic plaque, e.g., called a necrotic core fibroatheroma (NCFA) [see Nadkami, S. et al., Journal of Biomedical Optics 11(2):021006]. For example, NCFA, in simplest terms, can be described as a two layered tissue with a stiffer fibrous layer, rich in collagen and smooth muscle cells, overlying a deeper core of lower viscosity comprising lipid and necrotic debris. Based on Brownian motion considerations, the fibrous layer may affect a slower rate of speckle decorrelation (longer time constant) compared to the necrotic lipid layer (shorter time constant). Monte Carlo simulations and Diffusion theory studies have shown that as photons travel deeper into tissue, they have a higher probability of being remitted farther away from the illumination location, as shown in Figures 2 and 10(b). Due to this effect, in NCFA, when speckle decorrelation is measured as a function of radial distance, $\rho$, from the illumination location, a radially-dependent time constant, $\tau(\rho)$, may be observed. In proximity to the illumination location, most photons will only have traversed the stiff, high viscosity fibrous cap and the decorrelation times will be long. Farther away from the illumination site, the majority of photons will have propagated through the deeper low viscosity core and the decorrelation times will therefore be shorter, as shown in the illustrations 1010 and 1020 in Figures 10(a) and 10(b), respectively. The distance at which this time constant transition occurs can be correlated to the NCFA cap thickness.

To demonstrate this exemplary effect, it is possible to analyze spatio-temporal characteristics of LSI data obtained from an exemplary set of NCFAs. For each speckle image series, the position of the illumination spot, which can be approximately at the center of the plaque, may be manually located. Speckle decorrelation curves as a function of time can be obtained for each value of $\rho$, by performing a normalized 200 $\mu$m x 200 $\mu$m windowed cross-correlation centered at each $\rho$. Each window in the time series may be correlated with the first image window ($t$=0) in the Fourier domain. For each value of $\rho$ a normalized speckle decorrelation curve can be generated by extracting windowed cross-correlation maxima and normalizing them to the windowed autocorrelation maxima. The radially-resolved decorrelation time constant, $\tau(\rho)$, may be determined by exponential fitting of the decorrelation curve for each $\rho$, and by moving the center of the window in □□ increments of 50 $\mu$m. The window may be translated from the illumination spot to the ink mark locations for accurate registration with histology.

Due to tissue heterogeneity and variations in fibrous cap thickness, the measured laser speckle patterns may likely be asymmetric; hence, two graphs of $\tau(\rho)$ versus $\rho$ can be obtained which may correspond to either side of the illumination location for each NCFA. The distance, $\rho'$, at which $\tau(\rho)$ drops to half its maximum value at the illumination location, may be determined, as shown in Figure 10(a). Therefore, at distances smaller than $\rho'$, the observed speckle pattern can be predominantly affected by photon scattering within the fibrous cap (see Figure 10(b)). Conversely, for distances greater than $\rho'$, the observed speckle pattern can be primarily affected by scattering in the necrotic core.

Further, the radial distance, $\rho'$, can be associated with to the thickness of the fibrous cap, by combining a diffusion theory model of spatially-resolved diffuse reflectance and a Monte-Carlo model of light transport in tissue to estimate the radially-resolved maximum photon penetration depth through the NCFA fibrous cap. The tissue may be described by its optical parameters: the absorption coefficient, $\mu_\alpha$, the scattering coefficient, $\mu_s$, and the anisotropy coefficient, $g$, as well as the refractive indices of air and tissue ($n$ =1.4). For example, the optical properties of fibrous tissue can be derived at, e.g., about 632 nm by measuring the radially dependent remittance from an atherosclerotic plaque, histologically can be confirmed as a fibrous plaque. A fibrous plaque may be utilized, as the optical properties of collagen in a fibrous plaque may closely resemble the optical properties of fibrous caps in NCFAs.

Time-varying speckle images of the fibrous plaque may be obtained using the imaging set up as described above. Given the quantum efficiency and gain of the CCD camera, the total number of diffuse photons remitted from the plaque and detected by the CCD sensor may be measured by time-averaging speckle images acquired over a period of about 2 seconds. The radially-resolved photon probability, $P(\rho)$, for the fibrous plaque can be generated by summing the number of photons detected over different annuli of radii $\rho$, and then normalizing this value by the annulus area and the total number of photons detected over the area of the CCD detector. Next, the theoretical radial photon probabilities determined from a single-scatterer diffusion model for the case of a semi-infinite homogeneous tissue may be fitted to the measured radial photon probabilities, P(p), using an exemplary least-square optimization procedure, to extract the optical properties, $\mu_\alpha, \mu_s$ and $g$, of the fibrous plaque.

When the optical properties are established, they may be used as inputs to a Monte Carlo model which likely can assume a semi-infinite homogenous layer. Photon initial conditions can include input beams perpendicular to the semi-infinite layer. Multiple runs may be performed with the same or similar set of optical properties and a total of about 500,000 photon packet trajectories may be launched. Remitted photons may be collected over a radial distance of, e.g. about 2 mm. From the output of the Monte Carlo simulations, the radially-resolved maximum penetration depth may be recorded for each photon. The mean of the distribution of maximum penetration depths of photons remitted at a distance, $\rho$, can provide an estimate of the average maximum penetration depth, $z_{max}(\rho)$, as a function of radial distance. Additionally, for each plaque, $\rho'$ may be determined via spatio-temporal LSI as described above, and input into the $z_{max}(\rho)$ look up table to obtain a parameter, $z_{max}(\rho')$. The parameter, $z_{max}(\rho')$, obtained using the combined LSI - Monte Carlo technique for each NCFA can be compared with the fibrous cap thickness measured by histology using linear regression analyses and paired t-tests. For all analyses, a p-value < 0.05 can be considered statistically significant.

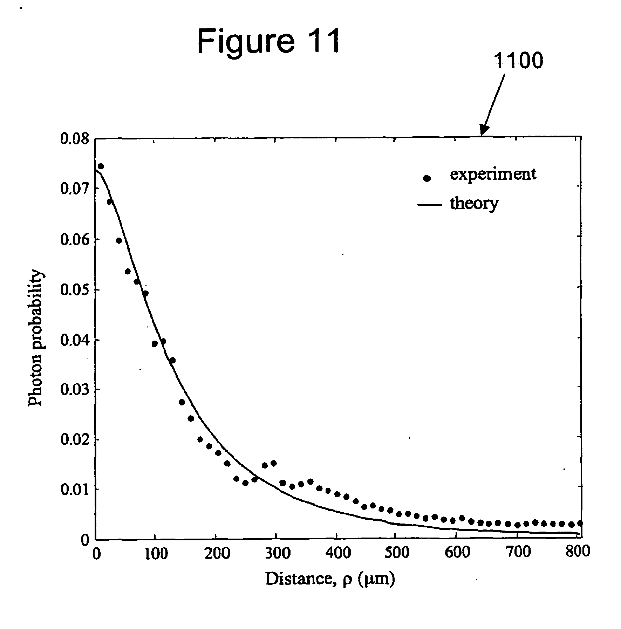

Figure 11 shows an a exemplary graph 1100 of the radial photon probabilities, $P(\rho)$, measured experimentally for the fibrous plaque compared with the theoretical radial photon probabilities calculated from the diffusion model for a semi-infinite homogenous tissue. The exemplary optical parameters may be obtained by utilizing a least squares optimization procedure to fit the theoretical curve to the experimental data yielded the following values for optical properties of the fibrous plaque: the absorption coefficient, $\mu_\alpha$ = 5.36cm$^{-1}$; the scattering coefficient, $\mu_s$ = 470.14cm$^{-1}$; and the anisotropy, g = 0.8. These values can correspond to previously published results of optical properties of the human aorta at about 632 nm wavelength.

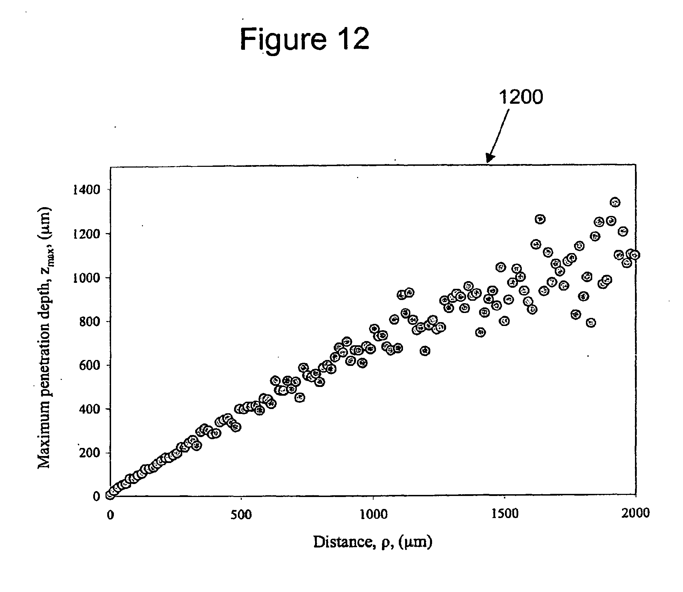

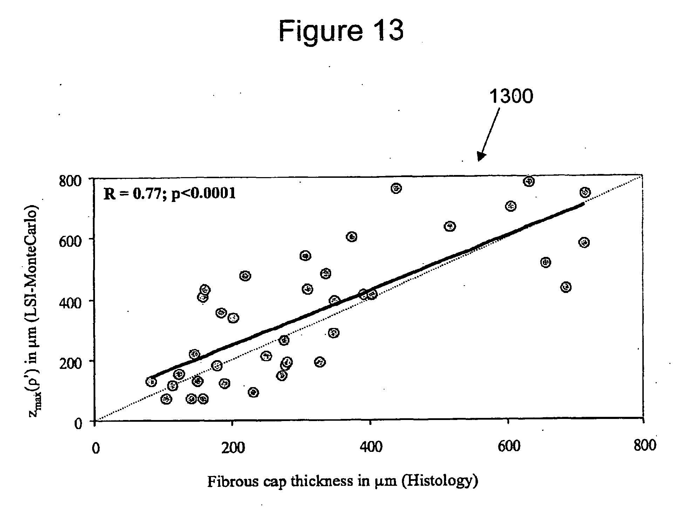

Figure 12 shows a graph 1200 of exemplary results of the Monte Carlo simulations in which the radially-resolved photon penetration depth, $z_{max}(\rho)$, is plotted as a function of distance, $\rho$, from the illumination location. The graph 1200 shows that the uncertainty in estimating $z_{max}(\rho)$ increases with distance from the illumination location. In Fig. 13, $z_{max}(\rho')$ provided in a graph 1300 versus the average fibrous cap thickness measured from histology. Two measurements of $\rho'$ and the corresponding parameter, $z_{max}(\rho')$ can be acquired, one for each side of the illumination location, and therefore a total of 38 measurements from 19 NCFAs may be obtained. Linear regression analyses demonstrated a strong positive correlation between $z_{max}(\rho')$ measured by the LSI-Monte Carlo technique and fibrous cap thickness ($R$ = 0.77, p < 0.0001). The exemplary results of the paired t-tests may indicated that there is likely no statistically significant difference between measurements of $z_{max}(\rho')$ made using our LSI - Monte Carlo technique and fibrous cap thickness measured from histological sections ($p$ = 0.2).

The foregoing merely illustrates the principles of the invention. Various modifications and alterations to the described embodiments will be apparent to those skilled in the art in view of the teachings herein. Indeed, the arrangements, systems and methods according to the exemplary embodiments of the present invention can be used with imaging systems, and for example with those described in International Patent Application PCT/US2004/029148, filed September 8, 2004, U.S. Patent Application No. 11/266,779, filed November 2, 2005, and U.S. Patent Application No. 10/501,276, filed July 9, 2004. It will thus be appreciated that those skilled in the art will be able to devise numerous systems, arrangements and methods which, although not explicitly shown or described herein, embody the principles of the invention and are thus within the scope of the present invention.

## Claims

1.   An apparatus for obtaining information regarding at least one portion of an anatomical structure, comprising:

at least one first arrangement which is configured to forward at least one first electromagnetic radiation to the at least one portion;
at least one second arrangement which is configured to detect at least one second electromagnetic radiation from the at least one sample, the at least one second electromagnetic radiation being related to the at least one first electromagnetic radiation;
at least one third arrangement which is configured to determine at least one motion characteristic as a function of depth within the at least one portion using the at least one second electromagnetic radiation, wherein the at least one third arrangement determines the at least one motion characteristic by obtaining data associated with the at least one second electromagnetic radiation, the data being speckle data detected by the at least one second arrangement; and **characterized by**
at least one fourth arrangement configured to receive the at least one first electromagnetic radiation and generate a plurality of third electromagnetic radiations to irradiate multiple points on the at least one portion, wherein the at least one second arrangement is configured to detect the at least one second electromagnetic radiation from the at least one sample based on the third electromagnetic radiations, and wherein the at least one third arrangement is further configured to determine a plurality of motion characteristics as a function of depth for a plurality of location of the at least one portion as a function of the at least one second electromagnetic radiation.

## Patentansprüche

1.   Eine Vorrichtung zum Erhalten von Information bezüglich zumindest eines Teils einer anatomischen Struktur, aufweisend:

zumindest eine erste Einrichtung, die zum Weiterleiten von zumindest einer ersten elektromagnetischen Strahlung zu dem zumindest einen Teil ausgeführt ist;
zumindest eine zweite Einrichtung, die zum Detektieren von zumindest einer zweiten elektromagnetischen Strahlung von der zumindest einen Probe ausgeführt ist, wobei die zumindest eine zweite elektromagnetische Strahlung mit der zumindest einen ersten elektromagnetischen Strahlung in Verbindung steht;
zumindest eine dritte Einrichtung, die zum Bestimmen von zumindest einer Bewegungseigenschaft als eine Funktion der Tiefe in dem zumindest einen Teil unter Verwendung der zumindest einen zweiten elektromagnetische Strahlung ausgeführt ist, wobei die zumindest eine dritte Einrichtung die zumindest eine Bewegungseigenschaft durch Erhalten von Daten, die mit der zumindest einen zweiten elektromagnetischen Strahlung in Verbindung stehen, bestimmt, wobei die Daten Speckledaten sind, die von der zumindest einen zweiten Einrichtung detektiert sind; und charakterisiert durch

zumindest eine vierte Einrichtung, die zum Empfang der zumindest einen ersten elektromagnetischen Strahlung und zum Generieren einer Mehrzahl von dritten elektromagnetischen Strahlungen zum Bestrahlen mehrerer Punkte auf dem zumindest einen Teil ausgeführt ist, wobei die zumindest eine zweite Einrichtung zum Detektieren der zumindest einen zweiten elektromagnetischen Strahlung von der zumindest einen Probe basierend auf den dritten elektromagnetischen Strahlungen ausgeführt ist, und wobei die zumindest eine dritte Einrichtung weiterhin zum Bestimmen einer Mehrzahl von Bewegungseigenschaften als Funktion der Tiefe für eine Mehrzahl von Orten des zumindest einen Teils als Funktion der zumindest einen zweiten elektromagnetischen Strahlung ausgeführt ist.

**Revendications**

1. Appareil pour obtenir des informations concernant au moins une partie d'une structure anatomique, comportant :

au moins un premier agencement qui est configuré pour transférer au moins un premier rayonnement électromagnétique vers la au moins une partie,
au moins un deuxième agencement qui est configuré pour détecter au moins un deuxième rayonnement électromagnétique provenant du au moins un échantillon, l'au moins un deuxième rayonnement électromagnétique étant associé au au moins un premier rayonnement électromagnétique,
au moins un troisième agencement qui est configuré pour déterminer au moins une caractéristique de mouvement en fonction de la profondeur dans l'au moins une partie en utilisant l'au moins un deuxième rayonnement électromagnétique, dans lequel le au moins un troisième agencement détermine l'au moins une caractéristique de mouvement en obtenant des données associées au au moins un deuxième rayonnement électromagnétique, les données étant des données de granularité détectées par l'au moins un deuxième agencement, et **caractérisé par**
au moins un quatrième agencement configuré pour recevoir l'au moins un premier rayonnement électromagnétique et générer une pluralité de troisièmes rayonnements électromagnétiques pour faire rayonner de multiples points sur la au moins une partie, dans lequel le au moins un deuxième agencement est configuré pour détecter le au moins un deuxième rayonnement électromagnétique provenant du au moins un échantillon sur la base des troisièmes rayonnements électromagnétiques, et dans lequel l'au moins un troisième agencement est également configuré pour déterminer une pluralité de caractéristiques de mouvement en fonction de la profondeur pour une pluralité d'emplacements de l'au moins une partie en fonction du au moins un deuxième rayonnement électromagnétique.

# Figure 1

100

# Figure 2

250
Modulated

230
Static

250
Modulated

Speckle
Pattern

200
|
Light in

210

$\rho$

Static Layer
(eschar)

220

Moving Layer
(perfused tissue)

205

240

Illuminate tissue with laser — 300

Obtain series of images as a function of time (t) — 310

Compute decorrelation function as a function of $\rho$ - distance from beam entry point — 320

Compute $\tau(\rho)$ — 330

Compute diffuse image by adding all images obtained in 320 — 340

Obtain optical properties ($\mu_a$, $\mu_s$ and g) from image obtained in step 330 — 350

Use Monte Carlo model with inputs $\tau(\rho)$ and optical properties to determine depth-resolved velocity profiles — 360

Figure 3

```
            ┌─────────────────┐
            │ Illuminate tissue│◄─────── 400
            │  with laser      │
            └─────────────────┘
                     │
                     ▼
        ┌──────────────────────────┐
        │ Obtain series of images taken│◄─────── 410
        │ with different exposure times T│
        └──────────────────────────┘
                     │
                     ▼
          ┌──────────────────┐
          │ Compute contrast │◄─────── 420
          │  of each image   │
          └──────────────────┘
                     │
                     ▼
      ┌──────────────────────────────┐
      │ Compute decorrelation function│◄─────── 430
      │  as a function of ρ - distance│
      │    from beam entry point      │
      └──────────────────────────────┘
                     │
                     ▼
            ┌──────────────────┐
            │ Compute τ(ρ)     │◄─────── 440
            └──────────────────┘
                     │
                     ▼
      ┌──────────────────────────────┐
      │ Compute diffuse image by adding│◄─────── 450
      │ all images obtained in Step 420│
      └──────────────────────────────┘
                     │
                     ▼
    ┌──────────────────────────────────┐
    │ Obtain optical properties (μₐ, μ_s and g)│◄─────── 460
    │   from image obtained in Step 430 │
    └──────────────────────────────────┘
                     │
                     ▼
    ┌──────────────────────────────────┐
    │ Use Monte Carlo model with inputs τ(ρ)│◄─────── 470
    │  and optical properties to determine│
    │    depth-resolved velocity profiles│
    └──────────────────────────────────┘
```

# Figure 4

# Figure 5

550

500

TRANSMITTER

LASER
DIODE

540

CCD

560

510

SPECIMEN

520

# Figure 6

# Figure 7

# Figure 8

# Figure 9

# Figure 10

# Figure 11

# Figure 12

# Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 01624401 A
- US 2004029148 W
- US 26677905 A
- US 50127604 A

### Non-patent literature cited in the description

- **STEWART CJ et al.** A comparison of two laser based methods for determination of burn scar perfusion: Laser Dopple versus laser speckel imaging. Elsevier, 2005
- **SADHWANI, A. et al.** *Appl. Opt.,* 1996, vol. 35 (28), 5727-5735
- **NADKARNI, S. et al.** *Journal of Biomedical Optics,* vol. 11 (2), 021006
- **THOMPSON, C.A.** *Appl. Opt.,* 1997, vol. 36, 3726
- **NADKAMI, S. et al.** *Journal of Biomedical Optics,* vol. 11 (2), 021006